# EUROPEAN PATENT APPLICATION

(11) **EP 4 424 677 A1**
(43) Date of publication of application: **04.09.2024**
(21) Application number: 23159988.7
(22) Date of filing: 03.03.2023
(51) Int. Cl.: C07D 303/32, C07F 9/113, C07C 43/315, C07F 9/10

(54) **PROCESS FOR THE SYNTHESIS OF (S)-EDELFOSINE OR (R)-EDELFOSINE**

(71) Applicant: RDP Pharma AG, 8590 Romanshorn (CH)
(72) Inventor: CHANG, Cheng-Hao, Taoyuan (TW); WANG, Yu Fa, Taoyuan (TW); MARTI, Roger, Fribourg (CH); MYLÈNE, Soudani, Fribourg (CH); CRISP, Antony, Romanshorn (CH)
(74) Representative: Forstmeyer, Dietmar

(57) **Abstract**

The present invention relates to an improved method for the synthesis of (S) or (R)-edelfosine ((S) or (R)-CP201).

## Description

The present invention relates to an improved method for the synthesis of (S) or (R)-edelfosine ((S) or (R)-CP201).

CP201 or edelfosine is a synthetic alkyl-lysophospholipid (ALP) having antineoplastic (anti-cancer) effects.

The present invention provides a method for the synthesis of (S)-edelfosine ((S)-CP201) comprising the following steps:

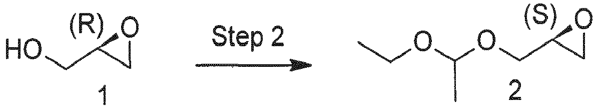

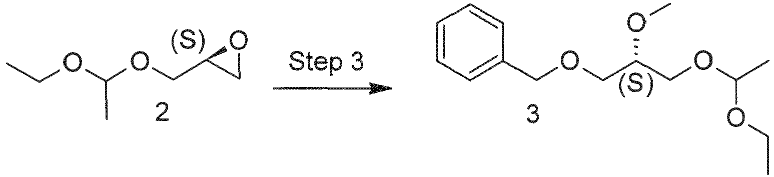

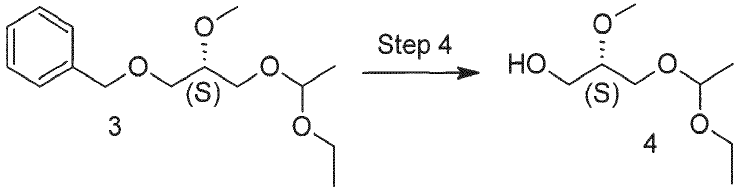

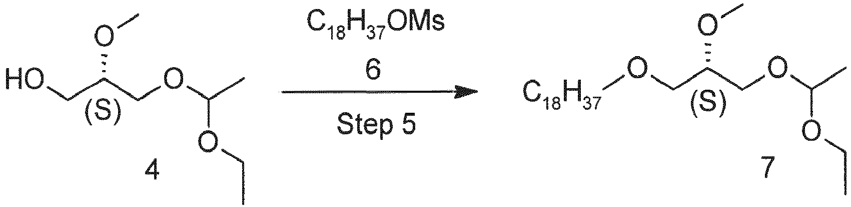

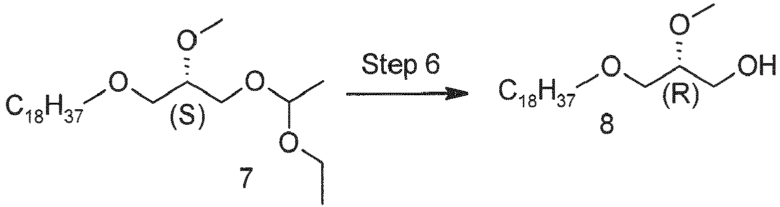

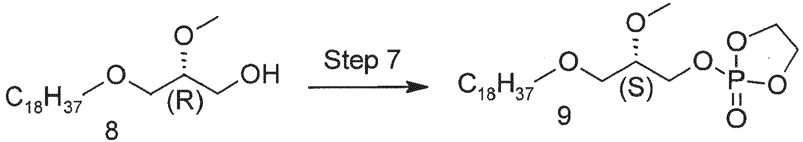

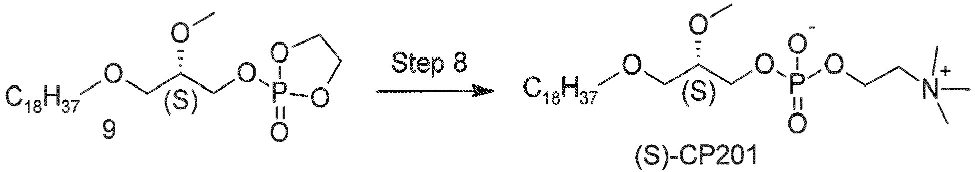

The present invention further provides a method for the synthesis of (R)-edelfosine ((R)-CP201) comprising the following steps:

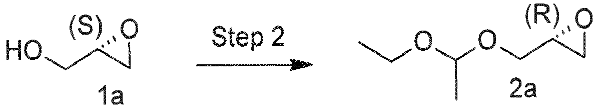

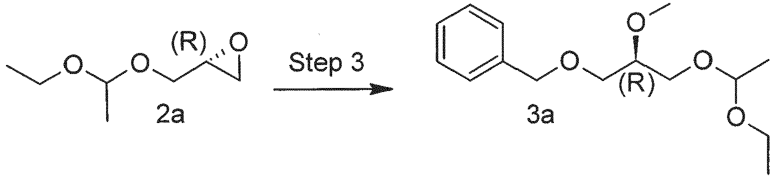

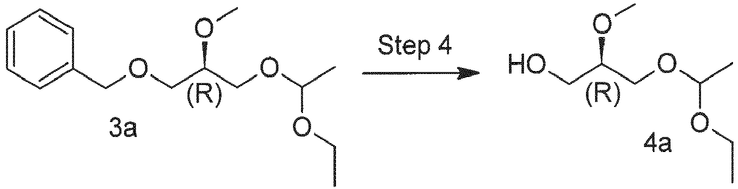

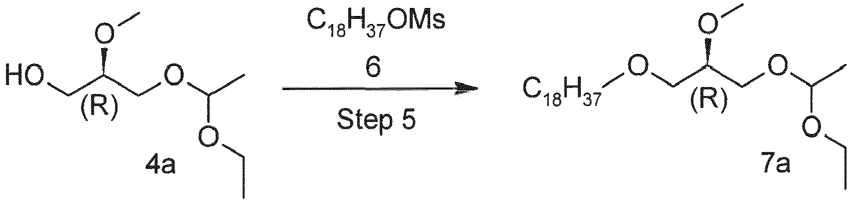

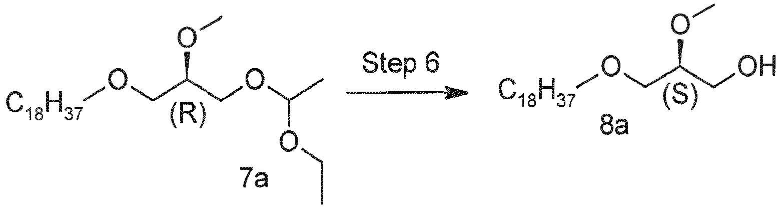

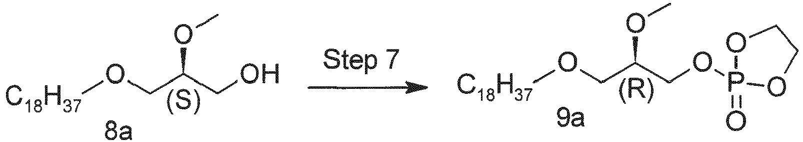

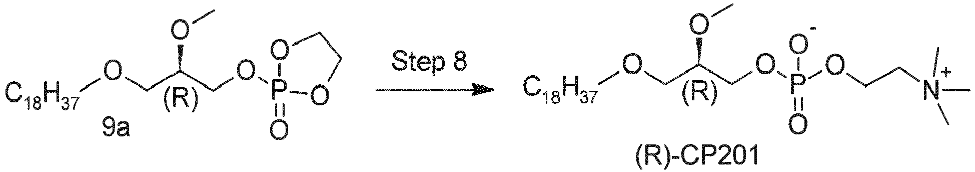

The present invention moreover provides a method for the synthesis of (R)-edelfosine ((R)-CP201) comprising the following steps:

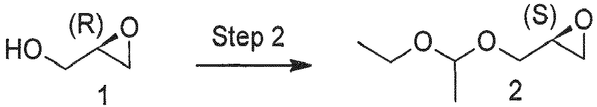

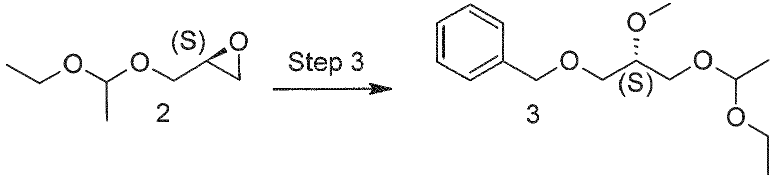

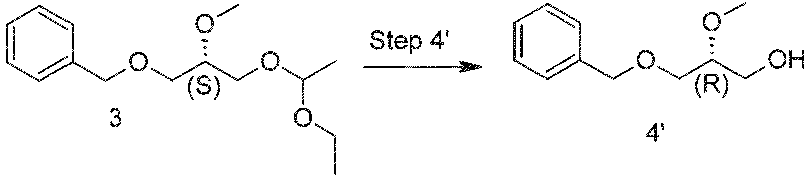

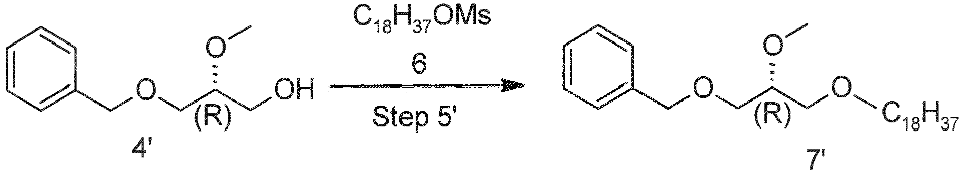

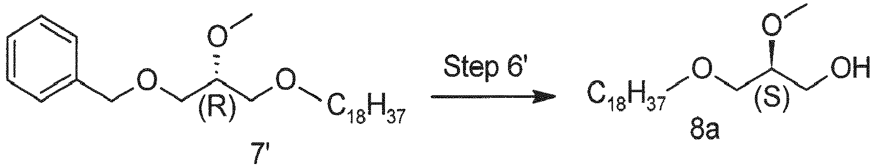

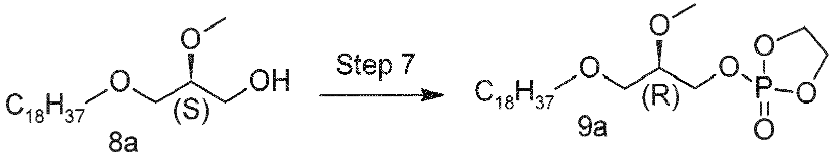

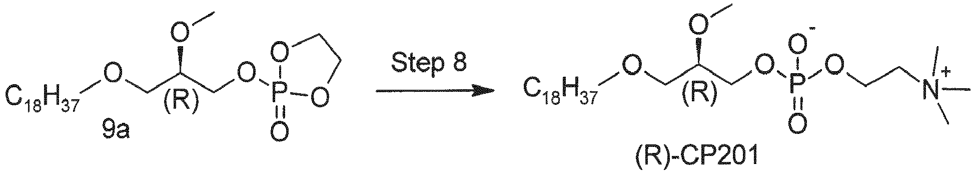

The present invention further provides a method for the synthesis of (S)-edelfosine ((S)-CP201) comprising the following steps:

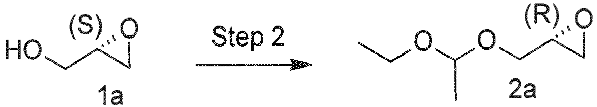

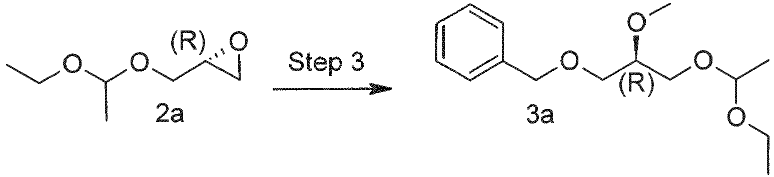

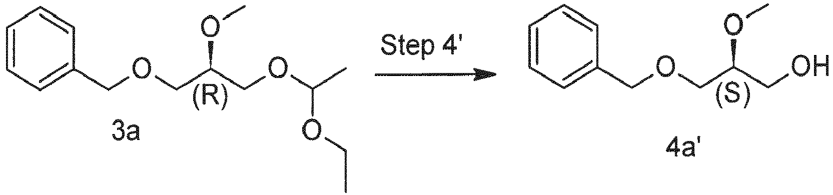

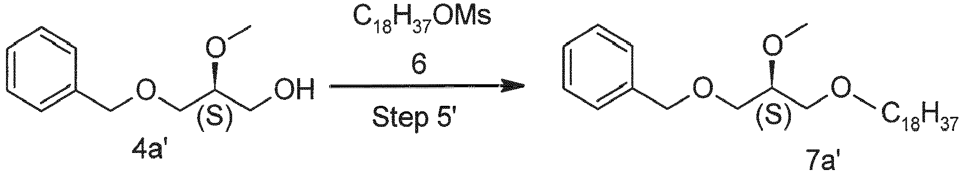

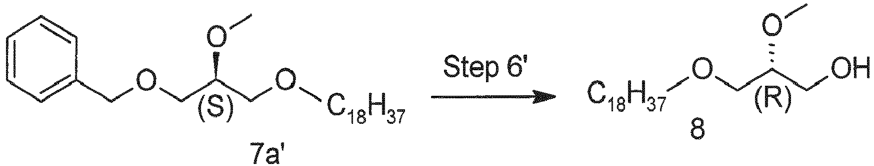

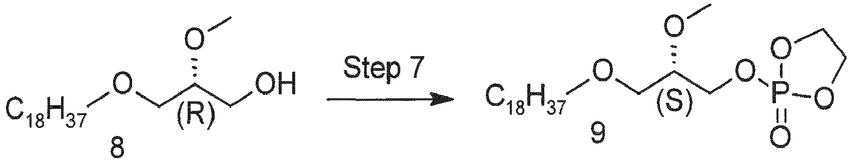

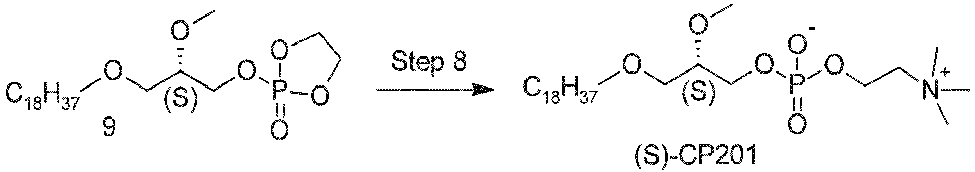

According to a preferred embodiment, step 2 is carried out in the presence of ethyl vinyl ether and p-toluenesulfonic acid and optionally a solvent.

Preferably, the optional solvent in step 2 is THF or toluene.

According to a further preferred embodiment, step 3 is carried out in the presence of benzyl alcohol, t-BuOK and optionally a solvent followed by the addition of methyl iodide.

Preferably, the optional solvent in step 3 is THF or toluene.

According to a further preferred embodiment, step 4 is carried out in the presence of hydrogen and Pd/C and EtOH.

According to a further preferred embodiment, step 5 is carried out in the presence of toluene and t-BuOK.

According to a further preferred embodiment, step 6 is carried out in the presence of methanol and methanesulfonic acid.

According to a further preferred embodiment, step 7 is carried out in the presence of pyridine/toluene, and 2-chloro-1,3,2-dioxaphospholane 2-oxide.

According to a further preferred embodiment, step 8 is carried out in the presence of trimethyl amine and acetonitrile.

According to a further preferred embodiment, compound 6 is prepared according to the following reaction:

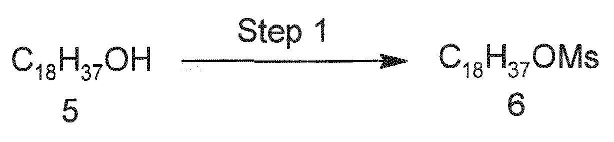

According to an especially preferred embodiment, step 1 is carried out in the presence of triethylamine and methanesulfonyl chloride and optionally a solvent (such as e.g., THF).

According to a further preferred embodiment in step 3 the following side product is obtained:

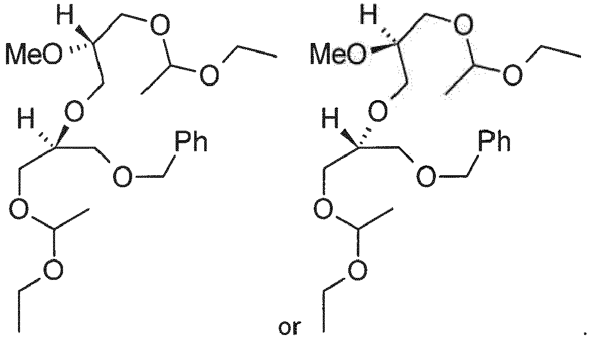

According to a moreover preferred embodiment in step 4 the following side product is obtained:

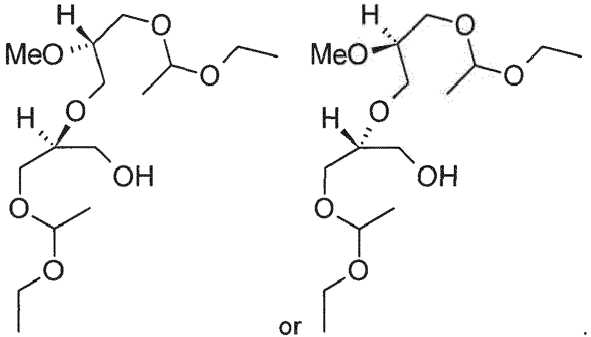

According to a further preferred embodiment, step 4' is carried out in the presence of acetic acid and a solvent (e.g. a mixture of butanol and water).

According to a further preferred embodiment, step 5' is carried out in the presence of toluene and t-BuOK.

According to a further preferred embodiment, step 6' is carried out in the presence of hydrogen and Pd/C and EtOH.

According to a further embodiment, the present invention is directed to (S)-edelfosine ((S)-CP201) which is obtainable by a process according to the present invention.

According to a further embodiment, the present invention is directed to (R)-edelfosine ((R)-CP201) which is obtainable by a process according to the present invention.

According to an especially preferred embodiment, ethyl vinyl ether is used as a protecting group in the synthesis of edelfosine (CP201).

According to a further embodiment, the present invention provides a compound which is selected from the following compounds:

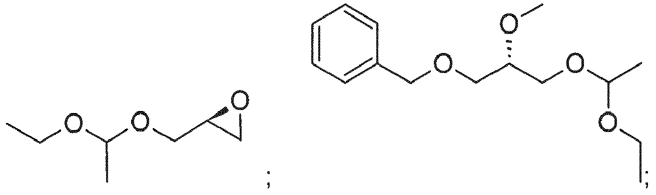

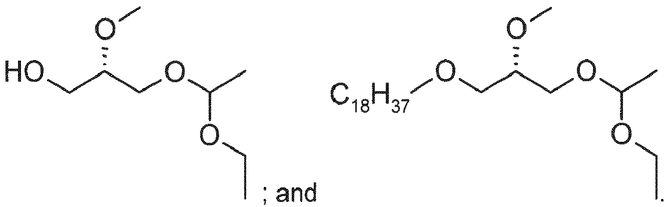

According to a further embodiment, the present invention provides a compound which is selected from the following compounds:

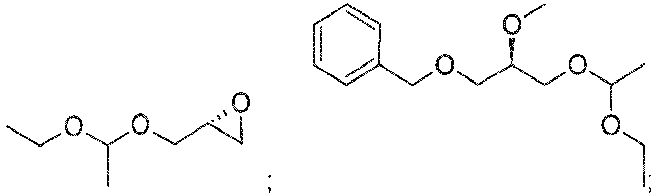

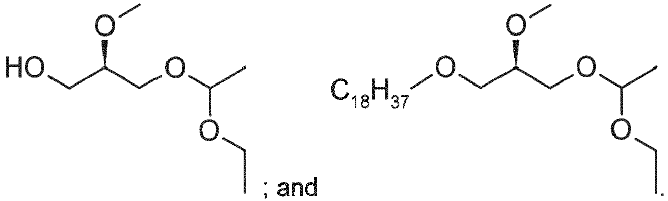

According to a further embodiment, the present invention provides a compound which is selected from the following compounds:

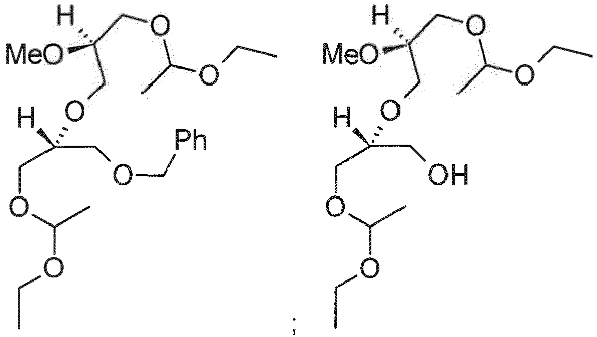

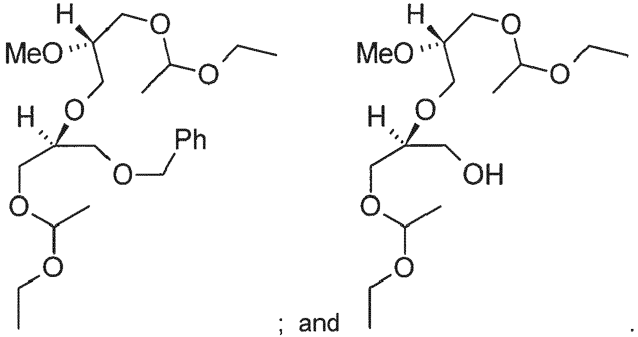

Table 1 shows a comparison of the yields between a previously applied synthesis process and the process of the present invention:

**Table 1:**

| Steps | Yield (%) | |
|---|---|---|
| | Previous process (protecting group: MOMCI) | Process of the present invention (protecting group: EVE) |
| Step 1 (to compound 6) | 90 | 96 |
| Step 2 (to compound 2) | 57 | 87 |
| Step 3 (to compound 3) | 84 | 88 |
| Step 4 (to compound 4) | 80 | 98 |
| Step 5 (to compound 7) | Combined to next step | |
| Step 6 (to compound 8) | 44 | 56 |
| Step 7 (to compound 9) | Combined to next step | |
| Step 8 (to (S)-CP201) | 68 | 49 |
| Total (except step 1) | 11 | 21 |

According to previous procedures, (S)-CP201 was synthesized in eight steps. During steps 2 to 6, the intermediate protection group MOM-Cl was used in the previous procedures. In contrast, the intermediate protection group EVE (ethyl vinyl ether) is used in the present invention.

Due to the different protection group, the overall yield (steps 2 to 8) increased from 11 %to21 %.

### Synthetic procedures

### 1. Preparation of compound 2

A three-neck round bottom flask equipped with a stirrer, nitrogen inlet and thermometer socket was charged with (R)-glycidol (90 g, 1.215 mol) in ethyl vinyl ether (450 mL, 5 vol) and p-toluenesulfonic acid (2.09 g, 0.012 mol). The reaction mixture was stirred for 2.5 hrs. at room temperature. Upon completion of the reaction (monitored by GC), 5% NaHCO₃ (180 mL) was added slowly to the reaction mixture. The temperature of the reaction mixture was kept below 25 °C. The organic layer was separated and the aqueous layer was extracted with DCM (2 × 90 mL). The combined organic layer was washed with water (90 mL), dried with MgSO₄ and concentrated under reduced pressure (< 30 torr) at 30°C to obtain crude compound 2 as yellowish oil. Crude compound 2 was purified by vacuum distillation at 50 to 60 °C using a high vacuum pump (about 5 torr) to afford compound 2 (154.14 g, yield: 86.8 %). ¹H NMR (300 MHz, CDCl₃) δ 4.77 (qd, *J* = 5.4 Hz, 1H), 3.85 - 3.38 (m, 4H), 3.19 - 3.12 (m, 1H), 2.81 (ddd, *J* = 5.1, 4.1, 1.0 Hz, 1H), 2.63 (ddd, *J* = 5.1 Hz, 1H), 1.33 (dd, *J* = 5.4, 4.1 Hz, 3H), 1.21 (td, *J* = 1.0 Hz, 3H). ¹³C NMR (75 MHz, CDCl₃) δ 99.68, 99.66, 65.75, 65.08, 60.94, 60.89, 50.91, 50.80, 44.63, 44.57, 19.75, 19.63, 15.26.

### 2. Preparation of compound 3

A four-neck round bottom flask, equipped with a stirrer, condenser, nitrogen inlet and thermometer socket, was charged with benzyl alcohol (110.961 g, 1.026 mol) in THF (500 mL, 10.0 vol) at room temperature. *t*-BuOK (134.327 g, 1.197 mol) was added over 10 min and the reaction mixture was heated at 60-65 °C and was stirred for 60 min. Compound 2 (50 g, 0.342 mol) in THF (250 mL, 5 vol) was added to the reaction mixture dropwise at 60-65 °C for 90 min and stirring was continued for 2 hrs. at 60-65 °C. The reaction mixture was allowed to cool to 25~30°C. Methyl iodide (194.191 g, 1.368 mol) was added to the reaction mixture dropwise at 25~40°C and the mixture was stirred overnight at 25~30°C. The progress of the reaction was monitored by GC; the analysis still showed unreacted BnOH. Another lot of t-BuOK (0.5 equiv) and Mel (0.5 equiv) was added to the reaction mixture dropwise at room temperature. Stirring of the reaction mixture was continued at room temperature. Upon completion of the reaction (monitored by GC), the reaction mixture was diluted with water (250 mL) and was extracted with MTBE (2 × 250 mL). The combined organic layer was washed with brine (250 mL) and was concentrated under reduced pressure (< 30 torr) at 45-50 °C to obtain compound 3 as an oil. The impurity of benzyl methyl ether was removed by vacuum distillation at 60-65 °C, using high vacuum (about 5 torr) to afford compound 3 (80.99 g, yield: 88.2 %).

### 3. Preparation of compound 4

A three-neck round bottom flask, equipped with stirrer and inlet hydrogen, was charged with compound 3 (87.62g, 0.327 mol) and 10% Pd/C (26.29 g, 30 % w/w) in 95% EtOH (613.34, 7.0 vol). The reaction mixture was stirred under H₂(g) (1 atm) and avoidance of light overnight at rt. Upon completion of the reaction (monitored by TLC), the reaction mixture was filtered through a celite bed and washed with 95% EtOH. (If TLC still shows compound 3, it is needed to add 10% Pd/C (5 % w/w) and stir overnight again). The organic layer was concentrated under reduced pressure (below 30 torr) at 45 °C. Crude compound 4 was obtained as yellowish oil (57.31 g, yield: 98.4 %). Before crude compound 4 was taken for the next step, azeotropy with toluene was carried out several times. ¹H NMR (300 MHz, CDCl₃) δ 4.72 (qd, *J* = 5.3 Hz, 1H), 3.84 - 3.38 (m, 10H), 2.21 - 2.08 (m, 1H), 1.32 (d, *J* = 5.3 Hz, 3H), 1.21 (td, 3H). ¹³C NMR (75 MHz, CDCl₃) δ 99.92, 80.10, 77.22, 63.88, 62.33, 61.20, 57.74, 19.69, 15.26.

### 4. Preparation of compound 6

A three-neck round bottom flask, equipped with a stirrer, condenser, nitrogen inlet and thermometer socket, was charged with compound 5 (60 g, 0.222 mol), THF (600 mL, 10 vol) and triethylamine (29.180 g, 0.288 mol) at 20~25 °C. Methanesulfonyl chloride (30.494 g, 0.266 mol) was added to the reaction mixture dropwise at below 30 °C. The reaction mixture was stirred for 4 hrs. at 25~30 °C. Upon completion of the reaction (monitored by HPLC), the reaction mixture was diluted with MTBE (300 ml) and washed with HCl (2 N) (300 ml). The MTBE layer was separated and the aqueous layer was extracted with MTBE (300 mL). The combined organic layer was washed with water (300 mL), aqueous sodium bicarbonate (300 mL), dried over MgSO₄ and concentrated under reduced pressure (below 20 torr) at 35~40 °C to afford compound 6 (74.35 g, yield: 96.0 %) as an off-white solid. ¹H NMR (300 MHz, CDCl₃) δ 4.22 (t, *J* = 6.6 Hz, 2H), 3.00 (s, 3H), 1.75 (dq, *J* = 6.6 Hz, 2H), 1.26 (s, 30H), 0.92 - 0.84 (m, 3H). ¹³C NMR (75 MHz, CDCl₃) δ 70.21, 37.38, 31.94, 29.71, 29.67, 29.62, 29.53, 29.44, 29.37, 29.14, 29.05, 25.43, 22.70, 14.13.

### 5. Preparation of compound 7

A four-neck round bottom flask, equipped with a stirrer, condenser, nitrogen inlet and thermometer socket, was charged with compound 4 (53 g, 0.297 mol), toluene (530 mL, 10 vol) and t-BuOK (36.7 g, 0.327 mol) at room temperature. The reaction mixture was stirred at 70-75 °C for 60 min. The reaction mixture was cooled to 30-35 °C. Compound 6 (83 g, 0.238 mol) was added to the reaction mixture at 30~35 °C. The reaction mixture was stirred for 2 hrs. at 100-105 °C. Upon completion of the reaction (monitored by HPLC), the reaction mixture was cooled to 25~30 °C, extracted with EA (1060 mL) and 10% NaCl_{(aq)} (530 mL). The aqueous layer was extracted with EA (530 mL) and the organic layer was filtered through a celite bed; then the organic layer was collected. MgSO₄ was added to the combined organic layer and the organic layer was concentrated under reduced vacuum, to obtain crude compound 7 (140.53 g). The crude product was charged with methanol (215 mL, 4 vol) and stirred for 30 min. The precipitated solids were filtered through a celite bed and washed with methanol. The methanol solution was directly taken for the next step.

### 6. Preparation of compound 8

A four-neck round bottom flask, equipped with a stirrer, condenser, nitrogen inlet and thermometer socket, was charged with compound 7 (114.72 g, 0.266 mmol) in methanol (265 ml, 2.31 vol) followed by methanesulfonic acid (2.56 g , 26.64 mmol) and the reaction mixture was heated at reflux for 1 h at 65~70 °C. Upon completion of the reaction (monitored by TLC), the reaction mixture was cooled to room temperature. The pH of the reaction mixture was adjusted to about 7 with saturated sodium bicarbonate solution (175 mL). The reaction mixture was concentrated and the residue was diluted with MTBE (575 mL). The solution was extracted with saturated sodium bicarbonate solution (115 mL) and pure water (115 mL). MgSO₄ was added to the organic layer and the organic layer was concentrated under reduced vacuum (< 30 torr) at 35°C, to obtain crude compound 8 (83.27 g). Crude compound 8 was purified by column chromatography: silica gel (1 kg), mobile phase: EA/hexane=1/5 to 1/3. Compound 8 was collected by TLC and concentrated under reduced vacuum (< 40 torr) at 35°C, to obtain compound 8 (59.73g, 56%). Before compound 8 was taken for the next step, azeotropy with toluene was carried out several times. ¹H NMR (300 MHz, CDCl₃) δ 3.82 - 3.20 (m, 10H), 2.16 (dd, 1H), 1.57 (d, 2H), 1.25 (s, 30H), 0.91 - 0.84 (m, 3H). ¹³C NMR (75 MHz, CDCl₃) δ 79.82, 71.96, 70.66, 62.73, 57.79, 31.94, 29.71, 26.09, 22.70, 14.13.

### 7. Preparation of compound 9

A three-neck round bottom flask, equipped with a stirrer, nitrogen inlet and thermometer socket, was charged with compound 8 (30 g, 0.084 mol) and toluene (300 mL, 10 vol). The reaction mixture was cooled to 5 °C and pyridine (9.926 g, 0.125 mol) was added, followed by 2-chloro-1,3,2- dioxaphospholane 2-oxide (17.878 g, 0.125 mol) which was dissolved in toluene (60 mL, 2 vol), at 5- 10 °C, dropwise. The reaction mixture was stirred for 3 hrs. at room temperature. Upon completion of the reaction (monitored by HPLC), the solid was filtered in a nitrogen bag; then the toluene layer was concentrated under reduced vacuum until 6 vol remained. The obtained compound 9 was directly taken for the next step.

### 8. Preparation of crude (S)-CP201

Trimethyl amine (74.177 g, 1.255 mol) in acetonitrile (485.45 ml, 15V) was added to compound 9 (38.87 g, 0.084 mol) in 97.55 ml toluene at room temperature in a serum bottle. The reaction mixture was stirred for 24 h at 65~70 °C, then cooled to room temperature and the precipitate was filtered under a nitrogen atmosphere and washed with acetonitrile. The solid was dried under reduced vacuum, to obtain crude (S)-CP201 (21.05 g, yield: 96.1%). ¹H NMR (300 MHz, CDCl₃) δ 4.31 (s, 1H), 4.08 - 3.71 (m, 4H), 3.59 - 3.11 (m, 16H), 2.49 (s, 7H), 1.53 (d, 2H), 1.25 (s, 28H), 0.95 - 0.81 (m, 3H). ¹³C NMR (75 MHz, CDCl₃) δ 77.22, 71.81, 57.81, 54.45, 31.94, 29.73, 29.69, 29.58, 29.38, 26.11, 22.70, 14.13.

### 9. Procedures of (S)-CP201 purification

### Stage 1. (Removing TMA and insoluble substance)

A mixture of crude (S)-CP201 (38.25 g, purity: 92-94%) and EA (10 vol) was stirred at 70°C for 30 min. The reaction mixture was cooled to room temperature. The solid was filtered and dried under high vacuum for 1 h to afford solid (S)-CP201 (37.84 g). IPA (9 vol) was added to the obtained solid and stirred at room temperature for 1 h. The turbid solution was filtered by filter paper several times. Further, the filtrate was filtered through a 0.2 µm membrane to receive a clear filtrate. The solvent was removed under reduced pressure and dried under high vacuum overnight to afford solid (S)-CP201 (36.95 g, yield: 60 %).

### Stage 2. (Increasing the purity level of (S)-CP201)

A mixture of solid (S)-CP201 (53.6 g) and 95% EtOH (1.2 vol) was stirred at 70°C. EA was added to the reaction solution and the temperature was kept at 70°C for 5-10 min. The reaction mixture was briskly cooled to 40-45 °C and stirred for 2 h. The reaction mixture was cooled to room temperature and stirred for 1 h. Further, the reaction mixture was cooled to 0-5 °C and stirred for 2 h. The solid was filtered and dried under high vacuum overnight to afford solid (S)-CP201 (46.91 g, yield: 87.5 %).

### Stage 3. (Removing the excess IPA)

A mixture of solid (S)-CP201 (59.24 g) and EA (10 vol) was stirred at 70°C for 1 h. The reaction mixture was cooled to room temperature and stirred for 3-4 h. Further, the reaction mixture was cooled to 0-5 °C and stirred for 1 h. The solid was filtered and dried at 33-35 °C overnight to afford a white powder of (S)-CP201 (57.24 g, yield: 96.62 %).

### Synthesis of (R)-edelfosine

(R)-edelfosine (or (R)-CP201) can be prepared in a similar manner as (S)-CP201 using (S)-glycidol as starting material.

### 10. Preparation of compound 4'

Compound 3 (0.54g, 1equiv) was engaged with acetic acid (1.4mL, 14 equiv) in a mixture of butanol and H₂O (3.2:1.9mL). The reaction was stirred at RT for 6 hours. The solution was quenched with NaOH 1M (some drop) and extracted with 2x10mL AE, then washed with brine (19mL). The crude product was purified by chromatography column. 0.26 g of desired product was recovered (Yield 76%; 99% purity).

### 11. Preparation of compound 7'

A three-neck round bottom flask was charged with t-BuOK (1.14g, 1.1equiv.), Compound 4' (1.787g, 1 equiv.) in toluene (10mL, 1M) at room temperature. The reaction mixture was stirred 70-75 °C for 60 min. Compound 6 (3.46g, 1.1equiv.) was added to the reaction mixture at 70-75°C. The reaction mixture was stirred for 3.5 hrs at 100-105 °C. Upon completion of the reaction (monitored by TLC), the reaction mixture was cooled to RT. AcOEt (10mL) and water (10mL) were added to the reaction mixture. The layers were separated. The aqueous layer was extracted with AcOEt (5x 10mL). Combined organics layers were washed with water (10mL) and brine (10mL). The org. layers were dried by Na₂SO₃, filtered and concentrated under vacuum. Crude product (3.8g) was purified by col. chrom (0-30% EA in Heptane in 10minutes then 20 minute at 30%). 3.48g of pure product was recovered (85% Yield; 97% Purity).

### 12. Preparation of compound 8a

A stirred solution of compound 7' (2.336g) in ethanol A15 5% IPA (15mL, 7Vol) was charged with 10 % Pd/C (Thermoscientific, 0.236g, 10%wt) in an autoclave. The reaction mixture was stirred under H₂ (2bar) for 6h at room temperature. Upon completion of the reaction (monitored by TLC), the reaction mixture was filtered through a celite bed and washed with EtOH (2 ×10mL). The organic layer was concentrated at 45 °C under reduced pressure to obtain compound 8a as grey solid (m=1.884g; 89% Yield; 89% Purity by qNMR). The product was directly taken for the next step.

The structures of all intermediate products and products were confirmed by ¹H-NMR, ¹³C-NMR and mass spectra.

## Claims

1. A process for the synthesis of (S)-edelfosine ((S)-CP201) comprising the following steps:

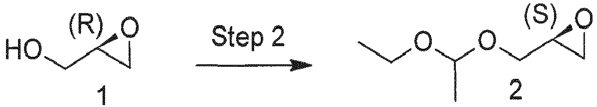

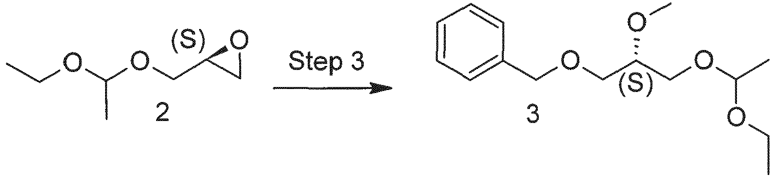

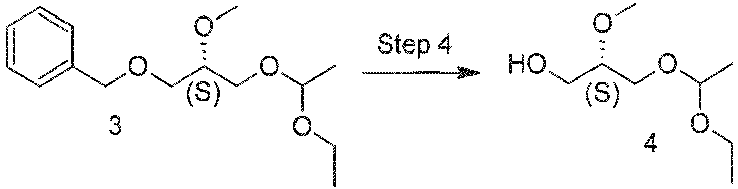

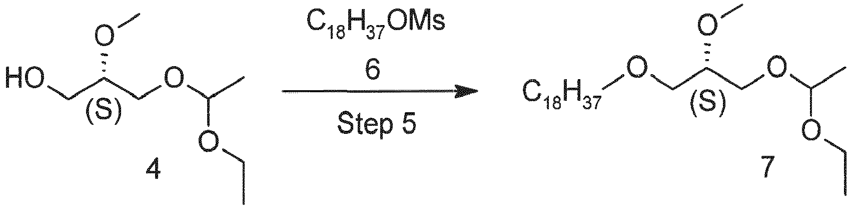

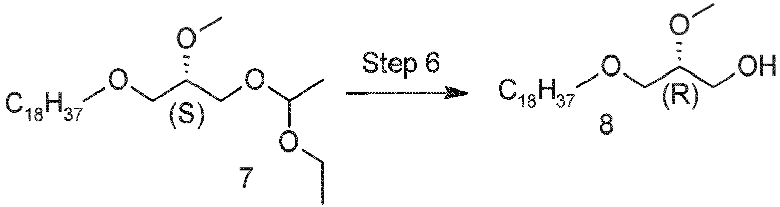

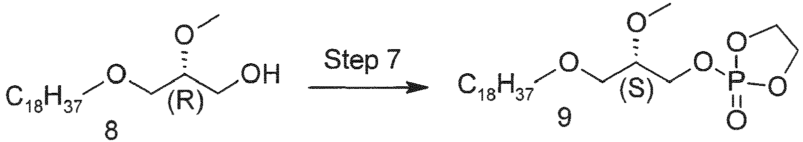

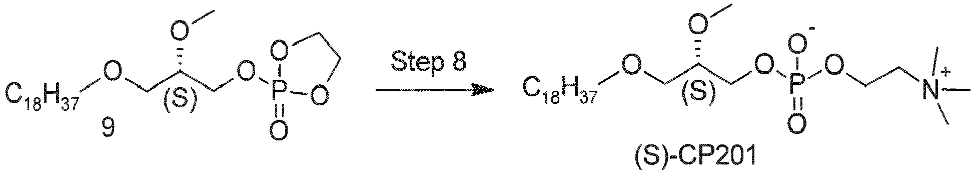

2. A process for the synthesis of (R)-edelfosine ((R)-CP201) comprising the following steps:

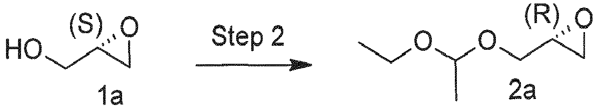

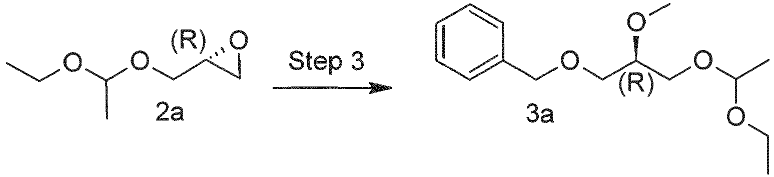

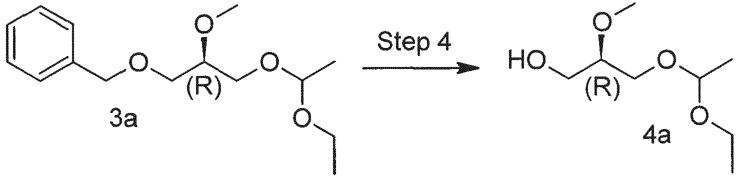

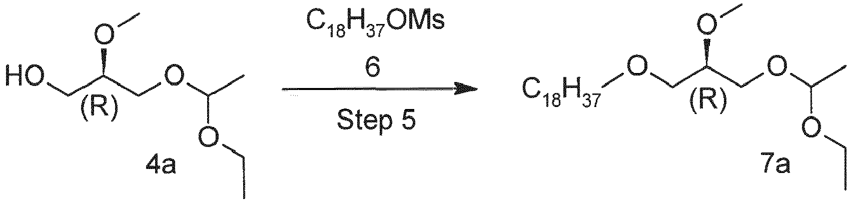

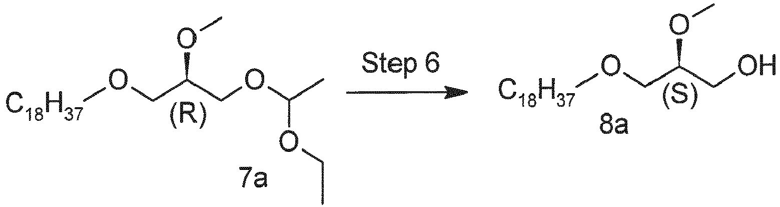

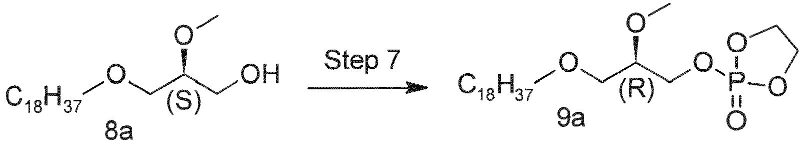

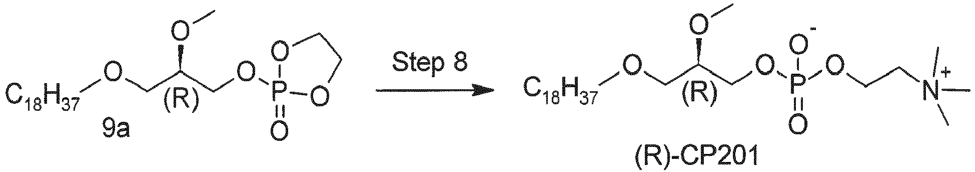

3. A process for the synthesis of (R)-edelfosine ((R)-CP201) comprising the following steps:

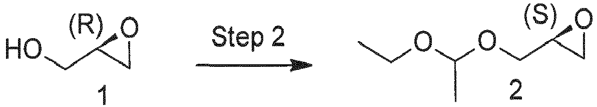

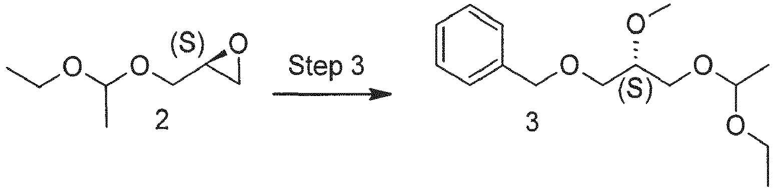

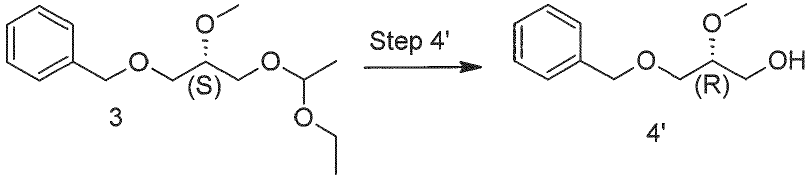

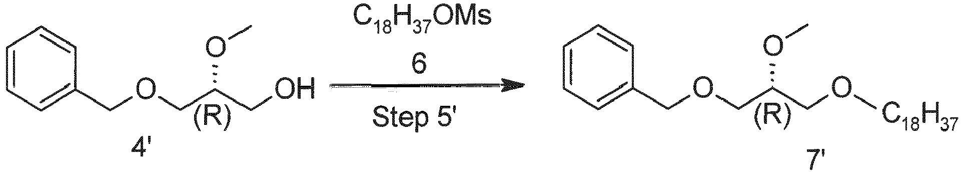

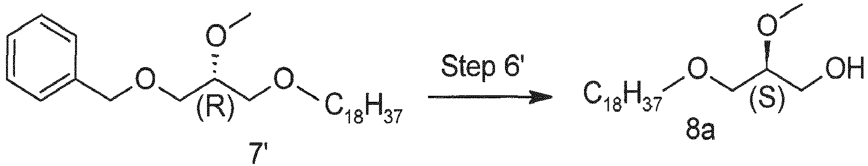

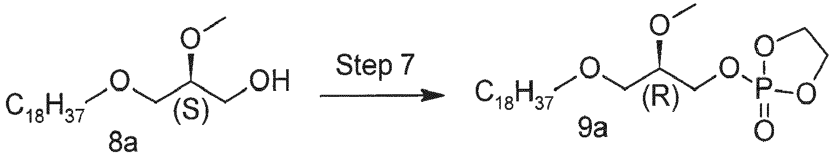

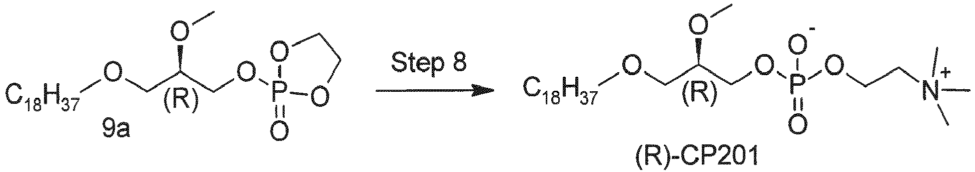

4. A process for the synthesis of (S)-edelfosine ((S)-CP201) comprising the following steps:

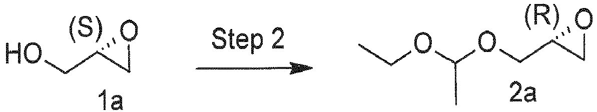

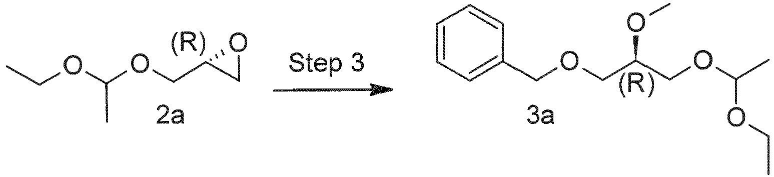

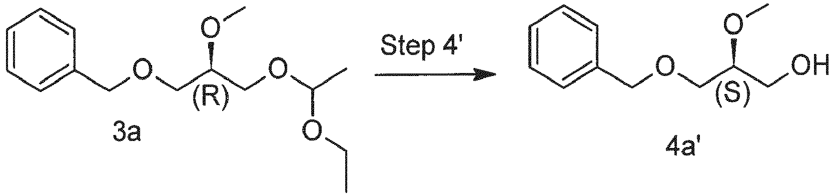

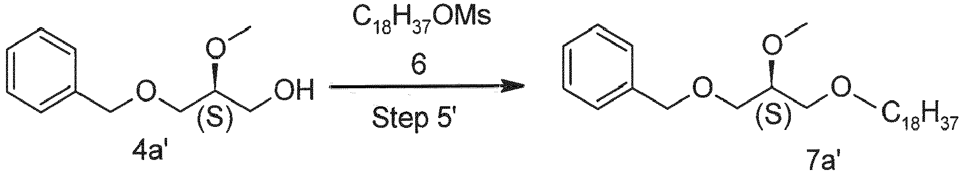

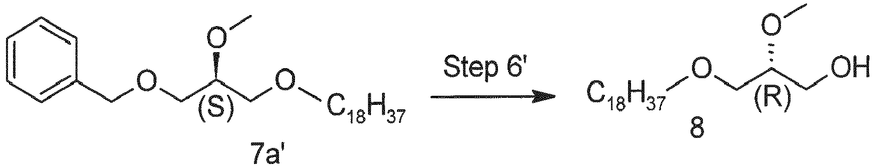

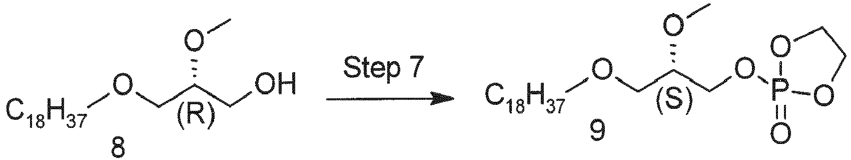

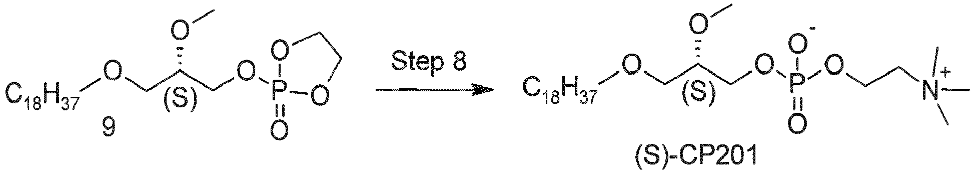

5. The process according to claim 1 or 2, wherein step 2 is carried out in the presence of ethyl vinyl ether and p-toluenesulfonic acid and optionally a solvent; and/or
wherein step 3 is carried out in the presence of benzyl alcohol, t-BuOK and optionally a solvent, followed by the addition of methyl iodide; and/or
wherein step 4 is carried out in the presence of hydrogen and Pd/C and EtOH; and/or
wherein step 5 is carried out in the presence of toluene and t-BuOK; and/or
wherein step 6 is carried out in the presence of methanol and methanesulfonic acid; and /or
wherein step 7 is carried out in the presence of pyridine/toluene, and 2-chloro-1,3,2-dioxaphospholane 2-oxide; and/or
wherein step 8 is carried out in the presence of trimethyl amine and acetonitrile.

6. The process according to claim 3 or 4, wherein step 2 is carried out in the presence of ethyl vinyl ether and p-toluenesulfonic acid and optionally a solvent; and/or
wherein step 3 is carried out in the presence of benzyl alcohol, *t*-BuOK and optionally a solvent, followed by the addition of methyl iodide; and/or
wherein step 4' is carried out in the presence of acetic acid and a solvent; and/or
wherein step 5' is carried out in the presence of toluene and t-BuOK; and/or
wherein step 6' is carried out in the presence of hydrogen and Pd/C and EtOH; and /or
wherein step 7 is carried out in the presence of pyridine/toluene, and 2-chloro-1,3,2-dioxaphospholane 2-oxide; and/or
wherein step 8 is carried out in the presence of trimethyl amine and acetonitrile.

7. The process according to claim 1 or 2, wherein in step 3 the following side product is obtained:

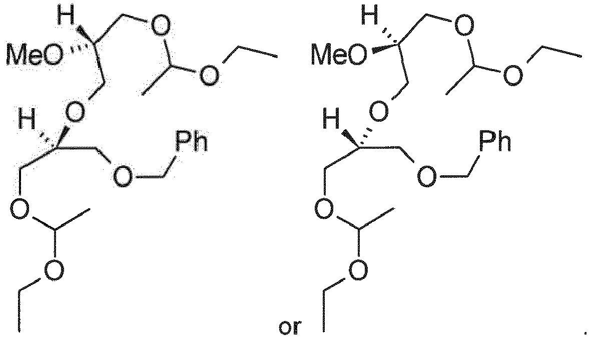

8. The process according to claim 1 or 2, wherein in step 4 the following side product is obtained:

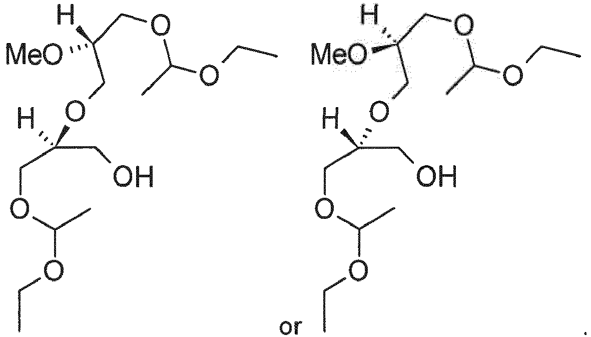

9. The process according to any one of the preceding claims, wherein compound 6 is prepared according to the following reaction:

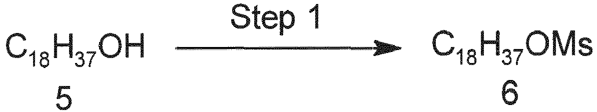

10. The process according to claim 9, wherein step 1 is carried out in the presence of triethylamine and methanesulfonyl chloride and optionally a solvent.

11. Use of ethyl vinyl ether as protecting group in the synthesis of edelfosine (CP201).

12. A compound selected from the following compounds:

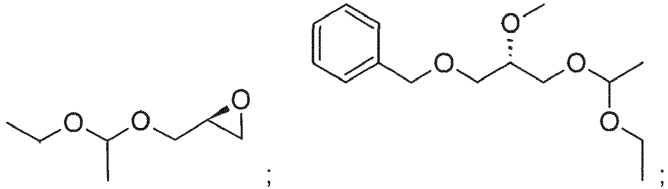

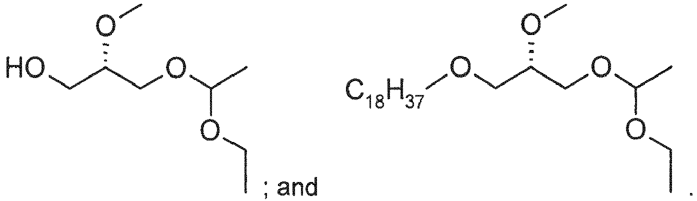

13. A compound selected from the following compounds:

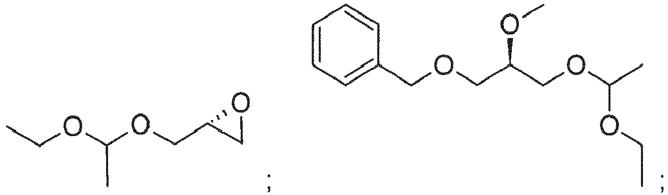

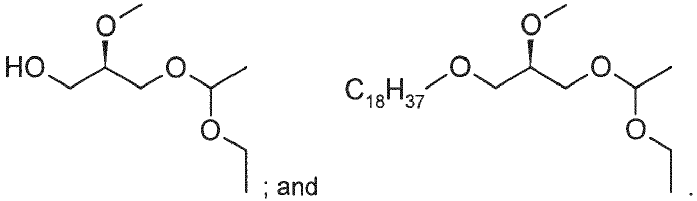

14. A compound selected from the following compounds:
